# EUROPEAN PATENT SPECIFICATION

(11) **EP 4 378 311 B1**
(45) Date of publication and mention of the grant of the patent: **01.04.2026**
(21) Application number: 22383160.3
(22) Date of filing: 30.11.2022
(51) Int. Cl.: A01N 47/44, A01N 31/02, A01N 25/02, A61K 31/155, A01P 1/00, A61K 31/07, A61K 31/10, A61K 31/12

(54) **METHOD TO PREPARE COLOURED CHLORHEXIDINE COMPOSITIONS**
VERFAHREN ZUR HERSTELLUNG VON GEFÄRBTEN CHLORHEXIDINZUSAMMENSETZUNGEN
MÉTHODE DE PRÉPARATION DE COMPOSITIONS COLORÉES DE CHLORHEXIDINE

(43) Date of publication of application: 05.06.2024
(73) Proprietor: Barna Import Medica, S.A., 08750 Molins de Rei (Barcelona) (ES)
(72) Inventor: Parés Rivero, Joan, 08750 Molins de Rei (Barcelona) (ES); Pérez González, José Antonio, 08750 Molins de Rei (Barcelona) (ES)
(74) Representative: Isern Patentes y Marcas S.L.

(56) References cited:
- CN-A- 104 771 781
- IT-A1- 201900 018 752
- US-A1- 2012 237 452
- US-A1- 2022 193 119
- LOUISE ARUP FISCHER ET AL: "Curcumin allergy in relation to yellow chlorhexidine solution used for skin disinfection prior to surgery", CONTACT DERMATITIS: ENVIRONMENTAL AND OCCUPATIONAL DERMATITIS, WILEY-BLACKWELL PUBLISHING, INC, US, vol. 51, no. 1, 3 August 2004 (2004-08-03), pages 39 - 40, XP071450608, ISSN: 0105-1873, DOI: 10.1111/J.0105-1873.2004.0378F.X

## Description

### FIELD OF THE INVENTION

The present invention relates to a method to prepare chlorhexidine antiseptic compositions for dermal/topical application in the form of solutions or dispersions.

### STATE OF THE ART

Chlorhexidine is a well-known topical antiseptic and disinfectant used for more than 30 years, having an international common accepted name, N,N"-Bis(4-chlorophenyl)-3,12-diimino-2,4,11,13-tetraazatetradecanediimidamide [also known as 1,6-di(4'-chlorophenyldiguanido)hexane]. Chlorhexidine has an empirical formula of C₂₂H₃₀Cl₂N₁₀ and a molecular weight of 505.45.

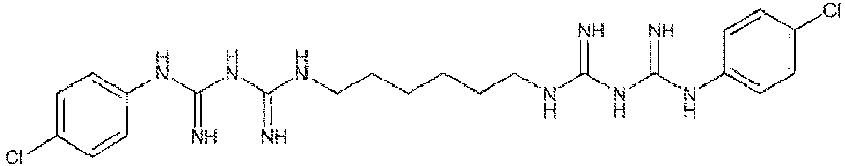

Most often chlorhexidine products are used for a number of applications such as daily hygiene application, oral antiseptic applications, hand and skin disinfection, coloured composition for topical disinfection etc. Chlorhexidine has been shown to be highly and broadly bactericidal, for instance against gram-positive and gram-negative as well as having an activity against yeasts, including *Candida albicans,* while having low levels of toxicity and strong skin binding properties.

The bactericidal activity of chlorhexidine differs from that observed with povidone-iodine or 70% isopropyl alcohol. Chlorhexidine has demonstrated an immediate bactericidal effect as well as a cumulative effect that persists for hours and even days after it is applied and, unlike iodophors, the germicidal activity of chlorhexidine has shown statistically significant efficacy in reducing bacterial counts in the presence of blood and other protein-rich biomaterials.

In many situations, it is highly relevant knowing where exactly the antiseptic has been applied. Thus, compositions for disinfecting areas of the skin are better suited if they are coloured, as to better identify the disinfected and treated areas. For instance, Louise Arup Fisher et al. ("Curcumin allergy in relation to yellow chlorhexidine solution used for skin disinfection prior to surgery", Contact dermatitis: environmental and occupational dermatitis, vol. 51, no. 1, (2004-08-23), pages 39-40, doi: 10.1111/J.0105-1873.2004.0378F.X) discloses the use of a chlorhexidine solution for skin desinfection prior to surgery, said solution comprising: 0.5% chlorhexidine gluconate, 83% ethanol (and 16.5% water) and 0.05% curcumin; the preparation method is not disclosed.

Chlorhexidine in solution is a non-coloured and clear liquid and, consequently, it is very difficult for the user to see where the liquid has been applied. However, numerous problems are encountered when colour, such as a tint or dye is added to an antiseptic solution of chlorhexidine in amount sufficient to stain a patient's skin when applied before, for instance, surgery. The tint or dye may react easily with the chlorhexidine causing it to be, to some extent, either precipitated or inactivated. Additionally, if some of the chlorhexidine becomes inactivated, the impurity profile increases, which affects the chemical stability of the product and may shorten the shelf life of the liquid composition.

An additional problem can arise if the colorant settles out of the liquid, as this may produce a nonuniform distribution of the coloured liquid when applied.

Chlorhexidine compositions comprising various colorants are known in the art. Previous attempts to use protected colorants in dermal applications were mostly carried out using different polymers or surfactants to aid the dissolution of colorants or dyes in the chlorhexidine composition.

US 2004/017989 acknowledges the above discussed problem associated with coloured chlorhexidine solutions and provides a solution by using a specialized applicator for applying chlorhexidine solution to a surface which comprises at least one ampoule formed of a frangible material and adapted to contain liquid to be applied; at least one hollow body defining an internal chamber adapted to receive at least one 50 ampoule; and at least one porous element that contains colorant, wherein the porous element is positioned such that liquid flows through the porous element when at least one ampoule is fractured and colorant is transferred to the liquid to be applied. However, it should be noted that the use of specific applicators to administer antiseptic solution, in many cases may result inconvenient or even incompatible with many compositions or with some applications.

US Patent Application Publication No. 2005/0265938 discloses decorative colour cosmetic compositions containing different pigment types and a carrier, wherein the different pigment types are physically separated from each other in individual capsules (silica capsules or micelles) within the carrier. The pigments remain separate and distinct in the final product and on the skin. The colour of the composition is determined by the relative amounts of the different pigments and remains the same upon application to the skin.

Later on, patent application WO 20071130981 disclosed a solution of chlorhexidine together with cationic dye in an amount sufficient to stain patient's skin. It is believed that as chlorhexidine pharmaceutically acceptable salts are cationic compounds, as such, are compatible with other cationic and non-ionic substances, but are chemically incompatible with anionic compounds. Furthermore, it should be noted that numerous disadvantages related with use of cationic dyes in antiseptic, have been later disclosed, for instance in EP 2 499 913 A1 formulation, these generally arosen from adverse toxicological and cancerogenic effects noticed.

US patent application US20120237452A1 also faces the problem of preparing coloured solutions comprising chlorhexidine, or a pharmaceutically acceptable salt thereof, which are safe and stable, avoiding the use of cationic dyes by using anionic dyes, but also solving the problems of incompatibility of anionic compounds with chlorhexidine and the associated precipitate formation. They provide a solution alternative to the addition of cationic excipients (e.g., cationic detergents, surfactants or excipients containing quaternary nitrogen) together with one or more anionic dyes to the chlorhexidine solution, which is the presence of a non-toxic solvent in the solution and the addition of the components in a particular order when the solution is prepared. The solvent can be water and about 20 to about 95% (v/v) of at least one C₁-C₈ lower alkanol such as isopropanol or ethanol. The process of preparing the solution consists of dissolving one or more anionic dyes in one or more first solvents to provide an anionic dye solution and adding chlorhexidine, or a pharmaceutically acceptable salt thereof to the anionic dye solution. A preferred process comprises the steps of dissolving one or more anionic dyes in deionized water to provide an anionic dye solution, then adding a portion of the amount of lower alkanol (preferably isopropanol), adding chlorhexidine or its salt previously dissolved. It is said that chlorhexidine can be added to the anionic dye solution is solid form but, preferably, it is added dispersed or dissolved in a solvent. The solvents used can be the same or different but with some limitations: if the solvent in the step where the anionic dye is dissolved is only water, chlorhexidine or its pharmaceutically acceptable salt must be added dissolved in a solvent which is not only water; if the solvent in the step where the anionic dye is dissolved is a mixture of water and isopropanol, chlorhexidine or its pharmaceutically acceptable salt must be added dissolved in a solvent which is only isopropanol, or only water or a mixture of isopropanol with water. US20120237452A1 shows assays carried out with chlorhexidine solutions comprising only one possible anionic dye, either Food Red 3 Carmoisine (which is also known as azorubine) or FD&C Red No. 40 (Allura Red). The composition containing carmoisine (azorubine) is prepared by dissolving said compound in ethanol (ethyl alcohol 70%) with stirring until complete dissolution, addition of chlorhexidine digluconate previously dissolved (20% v/w) in an aqueous solution, stirring until homogeneity and addition of more ethanol until obtaining 100 ml, obtaining a composition with 0.07% w/v carmoisine (azorubine), 0.2% w/v chlorhexidine digluconate and the quantity of ethyl alcohol (70%) necessary to complete 100 ml. Stability is monitored for up to 6 months, checking that the solution remains free of particles as well as the quantity of p-chloroaniline, which is 0.06% after 6 months of storage.at 25±2°C and 60%±5% relative humidity. The preparation of compositions comprising possible additional, non-anionic dyes, is not mentioned or considered.

US patent application US20220193119A1 also faces the problem of preparing aqueous antiseptic skin compositions comprising a cationic antiseptic (such as chlorhexidine or a pharmaceutically acceptable salt thereof such as chlorhexidine digluconate) and an anionic dye (also denominated anionic tinting agent in said application), but obtaining compositions which exhibit improved stability, good control and tinting ability. The solution is the addition of a polymer with polar binding sites wherein the polar site has a nitrogen carrying a positive charge and an oxygen carrying a negative charge, which is a high weight average molecular weight (at least 100,000 g/mol) polyelectrolyte; the polymer prevents or postpones the precipitation of the cationic antiseptic and the anionic colorant up to 24 months at 23 °C, possibly by forming complex with the anionic colorant and preventing the formation of insoluble precipitates cationic antiseptic - dye, The compositions provided contain, additionally to the at least one cationic antiseptic agent (preferably present in the range 0.1 - 2.5% by weight) and at least one anionic colorant (preferably in the range 0.01%-0.25% w/w, which is sufficient to tint the skin, carmoisine being one of the possible colorants), also one polymer or copolymer (for instance 0.1 - 10% w/w, being preferably polyvinyl pyrrolidone: PVP), at least one C₁-C₄ lower alcohol-based solvent (preferably at least 60% w/w, isopropanol, ethanol or n-propanol being preferred) and optionally a plasticizer. Several compositions comprising 0.05 % w/w carmoisine (azorubine), isopropanol (62.52 - 65.65% w/w), chlorhexidine digluconate (12.6% w/w) and water (19.96 - 21.6 % w/w) were tested (Table 2), being stable after 30 days only those also containing 2% w/w high molecular weight PVP; compositions comprising 0.05 % w/w carmoisine (azorubine), isopropanol (65.65% w/w), chlorhexidine digluconate (12.6% w/w), water (19.384 - 21.421 % w/w) and variable amounts of high molecular weight PVP (1.5, 2, 2.5, 3, 3.5 % w/w) and, additionally, 0.259 lactic acid 10%, are also disclosed in US20220193119A1. The process of preparing the skin antiseptic composition is disclosed as comprising: mixing until dissolved at least one anionic dye, cationic antiseptic and polymer with the lower alcohol solvent and water, which process is exemplified in Example 2 as consisting of dissolving the polymer in an ethanol/isopropyl alcohol and water mixture and adding chlorhexidine digluconate and carmoisine to the composition. The presence of additional, non anionic dyes is not considered.

EP2293761B1 also relates to complex chlorhexidine compositions for dermal/topical application. In this case, with chlorhexidine compositions comprising microencapsulated pigments to prevent them from precipitating, which when applied to the skin, such compositions produce a colour change indicating the delivery to the skin of the active substances contained in said compositions and a visual aesthetic.

Other compositions such those disclosed in WO 2017/200818, provided antimicrobial coatings for medical device articles, as for example urinary catheters, wherein said coating comprised a combination of chlorhexidine base (CHX), curcumin C3 complex (CUR) (which also comprises desmethoxycurcumin and bisdesmethoxycurcumin) and a silver salt (such as Ag sulfadiazine) a biomedical polymer with at least one solvent, such as THF in high concentrations. However, said compositions are not suitable for skin applications.

Chinese patent application CN104771781A discloses a liquid wound protection film comprising 70-80% w/w anhydrous ethanol, 4-6% w/w ethyl acetate, 3-5% butyl acetate, 2-4% w/w ethyl butyrate, 8-15% w/w cellulose acetate, 0.1-0.5% chlorhexidine, 0.5-1.5% w/w natural borneol; 0.1-5% w/w Panax notoginseng extract; 0.2-0.4% turmeric extract (comprising 5-6% curcumin) and 0.1-0.3% w/w dragon's blood extract. The composition does not comprise water. It is prepared by mixing anhydrous ethanol, ethyl acetate, butyl acetate and ethyl butyrate; adding cellulose acetate to the mixed solution, heating to 40°-50°C and stirring at low speed until the cellulose acetate is completely dissolved; adding chlorhexidine, natural borneol, Panax notoginseng extract, turmeric extract and Dragon's Blood extract to the mixed solution previously obtained in sequence; stirring to completely dissolve and, once the mixed solution is layered, filtering it and packaging it to obtain a finished liquid wound protection film.

Italian patent application IT201900018752A1 discloses a composition comprising: Eudragit RS 100 (a film forming polymer) 21.5%, *Curcuma longa* essential oil (turmeric, which comprises 5-6% curcumin) 2%, thyme essential oil 2%, cinnamon essential oil 2%, essential oil of oregano 2%; benzalkonium chloride 0.2%, chlorhexidine digluconate 0.3%, ethanol 96% balance. The composition comprises only 4% water and is for the treatment of periodontal pockets and peri-implant spaces.

There is a need to develop simple coloured chlorhexidine composition which overcomes the aforementioned problems wherein the chlorhexidine composition is, safe, stable overtime without being inactivated having a good impurity profile and providing a visible colour on the area of the patient where applied.

The present invention provides a solution to said problem.

### SUMMARY OF THE INVENTION

The invention is related to a method to prepare a topical antimicrobial aqueous composition, the composition comprising:
from 50-85% v/v of a C₁-C₆ linear or branched alcohol.
from 5-40% v/v of water;
from 0.001 % to 20 % w/v of di(4-chloro-phenyldiguanido) derivative of formula (1) 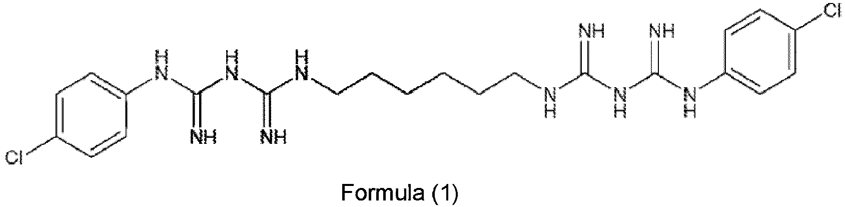
or a pharmaceutically acceptable salt thereof;
from 0.001 % to 1% w/v of curcumin;
which further comprises azorubine,

wherein the v/v and w/v percentages are based on the total volume of the composition,
   and
wherein the composition comprises less than 2 % of THF,
which method comprises at least the steps of:
   a) providing a solution of di(4-chloro-phenyldiguanido) derivative of formula (1) or a pharmaceutically acceptable salt thereof in water,
   b) providing a mixture of curcumin in water by adding water to a recipient with curcumin until dissolving it,
   c) providing a separate mixture for the additional component azorubine, by adding water to the recipient containing the additional component until dissolving it,
   d) adding to the mixture obtained in step a) the mixture of step b) and the mixture obtained in c),
   e) adding to the mixture of d) the quantity of water needed to reach the desired volume/volume percentage with regard to the total composition, while stirring at a temperature from 10 to 30 °C until homogenization,
   f) adding alcohol to the mixture of e) while stirring until homogenization.

### DETAILED DESCRIPTION OF THE INVENTION

The invention is related to a method to prepare a topical antimicrobial composition comprising:
from 50-85% v/v of a C₁-C₆ linear or branched alcohol.
from 5-40% v/v of water;
from 0,001 % to 20 % w/v of di(4-chloro-phenyldiguanido) derivative of formula (1) or a pharmaceutically acceptable salt thereof;
from 0.001 % to 1% w/v of curcumin;
which further comprises azorubine,

wherein the v/v and w/v percentages are based on the total volume of the composition, and
wherein the composition comprises less than 2 % of THF.

The above composition, as well as any possible embodiment thereof, will be considered a composition to be prepared or prepared in accordance with the method of the present invention.

The method of the invention to prepare the above composition provides a safe, stable and coloured composition for topical applications, providing a good visible bright orange colour on the skin of the patient to be applied.

The composition contains a significantly high amount of chlorhexidine or salts thereof without having to use additional solubilising components or other toxic excipients such as THF. This is an important advantage over the compositions disclosed, for instance, in international application WO 2017/200818, where the generally preferred compositions including curcumin contain 60 -90% v/v THF, which is used as solvent. THF is irritant to the skin and it is suspected to cause cancer after chronic exposure, so that compositions containing THF are not suitable for skin applications. The composition of the present invention, free of THF, have the advantage of being applicable to the body surface and particularly to the skin, while minimizing irritation, toxicological or cancerogenic risks in topical applications.

Moreover, the method of the invention has the advantage that the presence of curcumin and azorubine in the composition does not cause chlorhexidine to react giving rise, to some extent, that the chlorhexidine either precipitates or is inactivated. Additionally, the chlorhexidine composition prepared by the method of the invention is stable over time having a good impurity profile, thus providing a long shelf life of said composition.

Thus, the compositions prepared by the method of the invention do not need a separated compartment or pigment microencapsulation to prevent them to precipitate or react with other components of the composition, what makes them easier to prepare.

The term topical application refers to direct administration to a body surface, preferably to the skin. Thus, a topical composition is a composition intended for topical application/administration.

The term "stable" refers to a solution that after 24 hours is clear and leaves no residue solid, visible to the human eye. Moreover, the stable antiseptic solutions prepared by the method of the invention do not leave solids residues visible to the human eye after a stability test in the following conditions: Temperature 25 °C ±2°C & 60% HR, ±5% relative humidity during 1 month, preferably during 3 months, more preferably during 4.5 months and even more preferably during at least 30 months.

The stable antiseptic solutions prepared by the method of the invention may contain insubstantial amounts of the chlorhexidine degradation compound commonly determined to assess chlorhexidine stability in a product, p-chloroaniline, which is considered an impurity. Preferably, p-chloroaniline concentration will be less than 500 ppm (0.05% w/v), after having undergone the stability test above mentioned. In the assays shown in the Examples section, carried out with one of the preferred embodiments for the composition of the invention, p-chloroaniline values were inappreciable after 15 days of storage in the above mentioned conditions, were below 500 ppm (0.05%) after 1 month and that value was not exceeded even after 4.5 months.

In the context of the present invention the di(4-chloro-phenyldiguanido) derivative of formula (1) is the compound also known as chlorhexidine and as N,N"-Bis(4-chlorophenyl)-3,12-diimino-2,4,11,13-tetraazatetradecanediimidamide [also known as 1 ,6-di(4'-chlorophenyldiguanido)hexane], having an empirical formula of C22H30Cl2N1O and a molecular weight of 505.45 . The di(4-chloro-phenyldiguanido) derivative of formula (1) is represented by the formula:

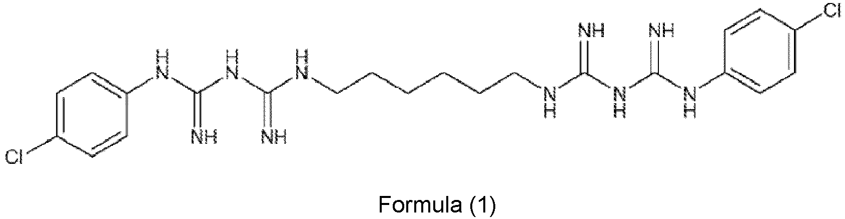

The term curcumin as used herein, means "curcuminoid", e.g., (E,E)-1,7-bis( 4-hydroxy-3 -methoxyphenyl)-1,6-heptadiene-3,5-dione or diferuloylmethane, its derivatives and preparations of the plant *Curcuma longa* or other curcumin-containing plants and having the basic general formula:

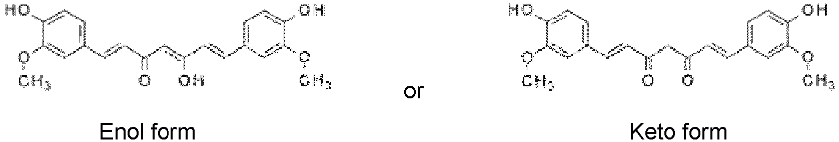

Curcumin is a tautomeric compound that exists in enolic form when dissolved in apolar solvents, and in keto form when dissolved in polar solvents.

In the context of the present invention, the term 'the composition is free of THF', is understood as the composition comprises less than 2% THF, Preferably the amount of THF in the composition is below 1%, more preferably below 0.05%.

Preferably, the composition is also free of fluoride ion from a fluoride ion source. In the context of the present invention, the term "composition free of fluoride ion from a fluoride ion source" means that the composition is free of fluoride ion from a fluoride ion source selected from the list consisting of fluoride, potassium fluoride, indium fluoride, stannous fluoride, and sodium monofluorophosphate and mixtures thereof. In the context of the present invention, the term "the composition is free of fluoride ion from a fluoride ion source" is understood as the composition containing less than from about 0.005% w/v based on the total volume of the composition.

The topical antimicrobial composition further comprises azorubine, which when used provides a dark orange to red colour and very visible and distinguishable on the skin. Thus, the colour may be adjusted to suit the different skin of different patients. Furthermore, the addition of this dye also provides stable compositions.

In a further preferred embodiment of the invention, the topical antimicrobial composition prepared by the method of the invention comprises azorubine in a percentage, from 0.001% to 1% w/v based on the total volume of the composition.

In another embodiment of the method of the invention, compatible with any other embodiment, the compositions comprise a C₁-C₆ linear or branched alcohol selected from the group consisting of: methanol, ethanol, propanol, isopropanol, 1-butanol, 2-butanol, 2-methyl-1-propanol, 2-methyl-2-propanol, 2-methyl-2-butanol,1-pentanol, 3-methyl-1-butanol, 2,2-dimethyl-1-propanol, 1-hexanol. More preferably, the C₁-C₆ linear or branched alcohol is ethanol, isopropanol or mixtures thereof. These two alcohols have similar values of density (ethanol: 0.789 g/cm³; isopropanol: 0.7863 g/cm³), so that similar amounts of any of the compounds give rise to similar proportions volume/volume in the composition and similar ranges of concentrations can be envisioned for them; moreover, their use, among other uses, as skin disinfectant or antiseptic compounds is well known, the use of ethanol in medicaments and cosmetic products being particularly frequent.

In another preferred embodiment of the method of the invention, also compatible with any other one, the composition comprises from 55-75% v/v of the C₁-C₆ linear or branched alcohols based on the total volume and/or from 20-45% by volume of water based on the total volume of the composition. Compositions with 55-75% v/v of a C₁-C₆ linear or branched alcohol, particularly those comprising isopropanol or ethanol (which are the preferred alcohols for the compositions to be prepared by the method of the invention), show a good spreading on the skin and good solubility of the components, such as of the chlorhexidine, the curcumin and the azorubine.

In a further preferred embodiment of the invention, the composition comprises the di(4-chloro-phenyldiguanido) derivative of formula (1) or the pharmaceutically acceptable salt thereof in a range from 0.1% to 10% w/v, preferably from 0.5 to 4% w/v, more preferably from 0,5% to 2% w/v or from 1% to 4% w/v, based on the total volume of the composition.

In another more preferred embodiment of the method of the invention, compatible with any other one, the pharmaceutically acceptable salt of the di(4-chloro-phenyldiguanido) derivative of formula (1) is the gluconate or the hydrochloride salt.

In another more preferred embodiment of the method of the invention, also compatible with any other one, the composition comprises curcumin in an amount from 0.01% to 0.5% w/v based on the total volume of the composition. Preferably, curcumin is present in an amount selected from the group of ranges of: 0.01% to 0.25% w/v, 0.01% to 0.20% w/v, 0.01% to 0.15% w/v, 0.01% to 0.12% w/v, 0.01% to 0.1% w/v, 0.01% to 0.085% w/v, 0.01% to 0.075% w/v, 0.01% to 0.065% w/v, 0.01% to 0.05% w/v, 0.01 to 0.025% w/v, 0.025% to 0.075% w/v and 0.025% to 0.05% w/v, all of them referred to the total volume of the composition. The range 0.01% to 0.12% w/v is particularly preferred, because curcumin concentrations in said range gives rise to clear liquid reddish solutions. Lower upper concentration limits, such as 0.050% w/v, can be also preferred due to the clearness of the solutions obtained while being still capable of providing colour on the body surface (skin) area where they are applied.

In another more preferred embodiment of the method of the invention, also compatible with any other one, the topical antimicrobial composition prepared further comprises excipients selected from the list consisting of: emollients, thickeners, preservatives, fragrances, pH regulators and gelling agents.

The composition prepared by the method of the invention is a composition which comprises azorubine, especially in the preferred range from 0.001% to 2% w/v based on the total volume of the composition. Thus, a preferred embodiment of the method of the invention, relates to the method to prepare a topical antimicrobial composition comprising:
from 50-85% v/v of a C₁-C₆ linear or branched alcohol,
from 5-40% v/v of water,
from 0.001 % to 20 % w/v of di(4-chloro-phenyldiguanido) derivative of formula (1) or a pharmaceutically acceptable salt thereof,
from 0.001% to 1% w/v of curcumin,
from 0.001 % to 2% w/v of azorubine
wherein the v/v and w/v percentages are based on to the total volume of the composition,
and optionally also comprising one or more pharmaceutically acceptable excipient(s) selected from the group of: emollients, thickeners, preservatives, fragrances, pH regulators and gelling agents.

Another particularly preferred embodiment of the method of the invention, relates to the method to prepare the composition where the ingredients are present in preferred ranges disclosed above. Thus, another particular preferred embodiment of the invention is a method to prepare a topical antimicrobial composition consisting of:
from 55-75% v/v of a C₁-C₆ linear or branched alcohol,
from 10-40% v/v of water,
from 1 % to 4 % w/v of di(4-chloro-phenyldiguanido) derivative of formula (1) or a pharmaceutically acceptable salt thereof,
from 0.01% to 0.075% w/v of curcumin,
from 0.001 % to 2% w/v of azorubine,
wherein the v/v and w/v percentages are based on the total volume of the composition,
and, optionally and additionally, also comprising one or more pharmaceutically acceptable excipient(s) selected from the group of: emollients, thickeners, preservatives, fragrances, pH regulators and gelling agents.

Yet a more particularly preferred embodiment of the method of the invention refers to the method to prepare a topical antimicrobial composition consisting of:
70% v/v isopropanol,
2% w/v gluconate salt of di(4-chloro-phenyldiguanido) derivative of formula (1);
0.05% w/v curcumin,
0.01 % w/v azorubine, and
q.s.p. (*quantitat sufficient per*) 100% v/v of water
wherein the v/v and w/v percentages are based on to the total volume of the composition,
and, optionally and additionally, one or more pharmaceutically acceptable excipient(s) selected from the group of: emollients, thickeners, preservatives, fragrances, pH regulators and gelling agents.

In another preferred embodiment of the method of the invention, the topical antimicrobial composition is an antimicrobial skin composition, thus suitable to be applied on the skin.

In an additional preferred embodiment of the method of the invention, compatible with all the previous ones, p-chloroaniline concentration in the topical antimicrobial composition prepared by the method of the invention is less than 0.05% w/v.

The invention relates to a method to prepare the topical antimicrobial composition disclosed above, in accordance to any of its possible embodiments, which comprises at least the steps of:
a) providing a solution of di(4-chloro-phenyldiguanido) derivative of formula (1) or a pharmaceutically acceptable salt thereof in water,
b) providing a mixture of curcumin in water by adding water to a recipient with curcumin until dissolving it,
c) providing a separate mixture for the additional component azorubine, by adding water to the recipient containing the additional component until dissolving it,
d) adding to the mixture obtained in step a) the mixture of step b) and the mixture obtained in c),
e) adding to the mixture of d) the quantity of water needed to reach the desired volume/volume percentage with regard to the total composition, while stirring at a temperature from 10 to 30 °C until homogenization,
f) adding alcohol to the mixture of e) while stirring until homogenization.

The above method is preferred in order to prepare the composition, as the final composition prepared by the method of the invention presents better stability and solubility. But other possible variants which are not part of the invention can be also used, provided that the limited solubility of curcumin in water is taken into account.

Preferably, the mixture of step a) is carried out until the di(4-chloro-phenyldiguanido) derivative of formula (1) or the pharmaceutically acceptable salt thereof is completely dissolved in water.

As the method of the present invention is a method for the preparation of the composition as defined in the method itself, the above disclosed preferences for the compositions are also preferred embodiments of the method of the invention, such as the preference for the gluconate salt or the hydrochloride salt of di(4-chloro-phenyldiguanido) derivative of formula (1) and/or for isopropanol or ethanol or mixtures thereof as the C₁-C₆ linear or branched alcohol, as well as for the preferred ranges for each component of the composition.

The low toxicity of curcumin and the very distinguishable colour provided allows the use of the composition prepared by the method of the present invention as good skin disinfectant for both standard patient applications and surgical applications.

The present invention will now be further illustrated by reference to the following Examples, which do not limit the scope of the invention in any way.

### EXAMPLES

The compositions of Examples 1 and 2 were prepared following the method above described as the preferred method of the present invention. The appropriate amount of each component was used in each case, as it is disclosed in the tables below. In said tables, the compound named "chlorhexidine gluconate" is the compound referred as the gluconate salt of the di(4-chloro-phenyldiguanido) derivative of formula (1) in previous sections of the present application.

### Example 1

### 1.1. Isopropanol Compositions: Initial observations

**Table 1a**

| **Component** | **Mixture 1a** | **Mixture 2a** | **Mixture 3a** | **Mixture 4a** |
|---|---|---|---|---|
| Chlorhexidine gluconate (g/100 ml) | 2 | 2 | 2 | 2 |
| Curcumin (g/100 ml) | 0.01 | 0.025 | 0.05 | 0.075 |
| Isopropanol 99.9% (mV100 ml) | 70 | 70 | 70 | 70 |
| Azorubine (g/100 ml) | 0.01 | 0.01 | 0.01 | 0.01 |
| Water (ml/100 ml) | ~30 (q.s.p. 100 ml) | ~30 (q.s.p. 100 ml) | ~30 (q.s.p. 100 ml) | ~30 (q.s.p. 100 ml) |

**Table 1b**

| **Component** | **Mixture 1b** | **Mixture 2b** | **Mixture 3b** | **Mixture 4b** | **Mixture 5b** | **Mixture 6b** |
|---|---|---|---|---|---|---|
| Chlorhexidine Gluconate (g/100 ml) | 2 | 2 | 2 | 2 | 2 | 2 |
| Curcumin (g/100 ml) | 0.15 | 0.20 | 0.25 | 0.30 | 0.35 | 0.5 |
| Isopropanol 99.9% (ml/100 ml) | 70 | 70 | 70 | 70 | 70 | 70 |
| Azorubine (g/100 ml) | 0 | 0 | 0 | 0 | 0 | 0 |
| Water (ml/100 ml) | ~30 (q.s.p. 100 ml) | ~30 (q.s.p. 100 ml) | ~30 (q.s.p. 100 ml) | ~30 (q.s.p. 100 ml) | ~30 (q.s.p. 100 ml) | ~30 (q.s.p. 100 ml) |

The compositions obtained were observed with the naked eye. Mixtures 1a to 4a were clear liquid orange-reddish solutions without solid residues visible to the human eye, while mixtures 1b to 6b were clear but show some undissolved particles on the bottom of the vial. Thus, all samples showed solubility, particularly good until 0.075g/100 ml of curcumin (0.075% w/v based on the total volume of the composition).

Mixture 3a was selected to perform the stability test over time.

Additionally, it was decided to carry out a supplementary assay to have a more accurate determination of the curcumin saturation concentration

### 1.2. Saturation assays

An assay was carried out to compare the curcumin saturation on different sample concentrations in chlorhexidine 2% w/v concentrations. 12 sample were prepared, 6 with isopropanol as alcohol (mixtures 1p-6p, with the same formulation as those of Table 1b) and 6 with ethanol as alcohol (mixtures 1e-6e), as follows:

**Table 2**

| **Component** | **Mixture 1p/1e** | **Mixture 2p/2e** | **Mixture 3p/3e** | **Mixture 4p/4e** | **Mixture 5p/5e** | **Mixture 6p/6e** |
|---|---|---|---|---|---|---|
| Chlorhexidine Gluconate (g/100 ml) | 2 | 2 | 2 | 2 | 2 | 2 |
| Curcumin (g/100 ml) | 0.15 | 0.20 | 0.25 | 0.30 | 0.35 | 0.5 |
| Isopropanol 99.9% / ethanol 99.9% (ml/100 ml) | 70 | 70 | 70 | 70 | 70 | 70 |
| Azorubine (g/100 ml) | 0 | 0 | 0 | 0 | 0 | 0 |
| Water (ml/100 ml) | ~30 (q.s.p. 100 ml) | ~30 (q.s.p. 100 ml) | ~30 (q.s.p. 100 ml) | ~30 (q.s.p. 100 ml) | ~30 (q.s.p. 100 ml) | ~30 (q.s.p. 100 ml) |

As commented in section 1.1, mixtures 1p-6p were liquid clear, orange-redish solutions with some little deposits on the bottom of the vials, while mixtures 1e-6e were liquid clear, more intense red solutions, also with some deposits on the bottom of the vials.

The assays were performed by HPLC, in a Kromasil 100-5-C18 column of 4.6x250mm, GI-C-193. The conditions were as follows: detector, PDA UV 254 nm; flow rate 1.0 ml/min; injection volume 1 µl; column oven at 30°C; autosample temperature 15°C, run time 20 min, adquisition time 17 min, mobile phase A: H₂O-0.1% TFA (trifluoroacetic acid); mobile phase B: ACN (acetonitrile)-0.1% TFA. The gradient of mobile phase A : mobile phase B along time was: 0.00 min, A: 60, B:40; 10.00 min A: 10, B: 90; 10.10 min: A: 60, B: 40; 17.00 min: A: 60, B:40. Curcumin (Docuchem, Girona, Spain) was used as reference standard.

Results of HPLC analysis show that saturation point of curcumin in chlorhexidine 2% w/v formulation is achieved around 0.12% w/v in the formulations with H₂O:isopropanol 30:70, and at 0.122% w/v in the formulation with H₂O:ethanol 30:70 (wherein all w/v percentages are based on the total volume of the prepared formulation).

Based on the above experiments (those of sections 1.1 and 1.2), it was concluded that the curcumin concentration range of 0.01% w/v - 0.12% w/v based on the total volume of the composition is a suitable range for the amount in curcumin in the composition, both when ethanol or when isopropanol is the alcohol present.

### Example 2

### 2.1. Stability test with isopropanol compositions

A stability test over time was performed with Mixture 3a of section 1.1. Several samples of Mixture 3a were prepared and were maintained at a temperature of 25°C and 60% relative humidity (RH) during different time periods: 0 days (sample analyzed the same day it was prepared), 15 days, 1 month, 2 month, 4.5 months.

At each indicated time point, samples were analyzed by HPLC, using chlorhexidine acetate as standard (Docuchem, Girona, Spain). HPLC analysis was performed by using conventional C18-column (150 m × 3.0 mm inner diameter) in flow rate of 0.5 mL/min under detection of diode array detector at 241 nm wavelength (Agilent 1200 SERIES HPLC System; Agilent Technologies). Column oven temperature was set to 25°C, and injection volume was 20 µl.

The results are shown in Table 3 below.

**Table 3**

| **Parameters to test** | **Time 0** | **15 days** | **1 month** | **2 months** | **4.5 months** |
|---|---|---|---|---|---|
| Appearance | Clear reddish solution with no precipitate or turbulence | Clear reddish solution with no precipitate or turbulence | Clear reddish solution with no precipitate or turbulence | Clear reddish solution with no precipitate or turbulence | Clear reddish solution with no precipitate or turbulence |
| Total impurities % w/v | <1%% | <1% | <1% | <1% | <1% |
| p-chloroaniline | N/A* | N/A* | Below 500 ppm | Below 500 ppm | Below 500 ppm |

| | | | | | |
|---|---|---|---|---|---|
| *N/A: Not appreciable | | | | | |

A similar experiment carried out at 30°C and 60% RH gave rise to similar values of p-chloroaniline.

Thus, the samples, surprisingly, showed good stability over time since, even 4.5 months after preparation, total impurities remain below 1 % w/v and p-chloroaniline did not exceed the value of 500 ppm (0.05% w/v). It is expected that said value will not be exceeded within the period of 30 months after preparation.

As the compositions prepared with isopropanol or ethanol show good solubility, good stability and a good impurity profile over time, they can be considered suitable to be used as coloured antiseptic compositions.

## Claims

1. A method to prepare a topical antimicrobial aqueous composition,
the composition comprising:
from 50-85% v/v of a C₁-C₆ linear or branched alcohol.
from 5-40% v/v of water;
from 0.001 % to 20 % w/v of di(4-chloro-phenyldiguanido) derivative of formula (1) 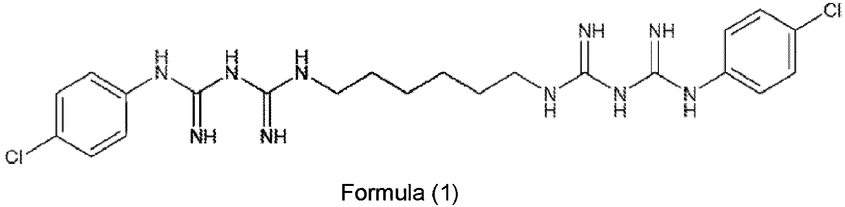
or a pharmaceutically acceptable salt thereof;
from 0.001% to 1% w/v of curcumin;
which further comprises azorubine,
wherein the v/v and w/v percentages are based on the total volume of the composition,
and wherein the composition comprises less than 2 % of THF;
which method comprises at least the steps of:
a. providing a solution of di(4-chloro-phenyldiguanido) derivative of formula (1) or a pharmaceutically acceptable salt thereof in water,
b. providing a mixture of curcumin in water by adding water to a recipient with curcumin until dissolving it,
c. providing a separate mixture for the additional component azorubine, by adding water to the recipient containing the additional component until dissolving it,
d. adding to the mixture obtained in step a) the mixture of step b) and the mixture obtained in c),
e. adding to the mixture of d) the quantity of water needed to reach the desired volume/volume percentage with regard to the total composition, while stirring at a temperature from 10 to 30 °C until homogenization,
f. adding alcohol to the mixture of e) while stirring until homogenization.

2. The method according to claim 1, wherein the azorubine is present in the composition from 0.001% to 1% w/v based on the total volume of the composition.

3. The method according to any of the preceding claims, wherein the C₁-C₆ linear or branched alcohol is isopropanol or ethanol.

4. The method according to any of the preceding claims, wherein the volume percentage of the C₁-C₆ linear or branched alcohol is from 55-75% v/v and/or the volume percentage of water is from 20-45% v/v based on the total volume of the composition.

5. The method according to any of the preceding claims, wherein the di(4-chloro-phenyldiguanido) derivative of formula (1) or the pharmaceutically acceptable salt thereof is present in the composition in a range from 0.1 to 10% w/v based on the total volume of the composition.

6. The method according to claim 5, wherein the di(4-chloro-phenyldiguanido) derivative of formula (1) or the pharmaceutically acceptable salt thereof is present in the composition in a range from 1 to 4% w/v, based on the total volume of the composition.

7. The method according to any one of the preceding claims, wherein the pharmaceutically acceptable salt of the di(4-chloro-phenyldiguanido) derivative of formula (1) is the gluconate or the hydrochloride salt.

8. The method according to any one of the preceding claims, wherein the amount of curcumin is from 0.01% to 0.5% w/v based on the total volume of the composition.

9. The method according to claim 8, wherein the amount of curcumin is from 0.01% to 0.12% w/v based on the total volume of the composition.

10. The method according to any one of the preceding claims, wherein the composition further comprises excipients selected from the list consisting of emollients, thickeners, preservatives, fragrances, pH regulators and gelling agents.

11. The method according to any one of the preceding claims, comprising:
70% v/v isopropanol,
2% w/v gluconate salt of di(4-chloro-phenyldiguanido) derivative of formula (1);
0.050% w/v curcumin,
0.01 % w/v azorubine, and
q.s.p. 100% v/v of water
wherein the percentages are based on the total volume of the composition,
and, optionally also comprising one or more pharmaceutically acceptable excipient(s) selected from the group of: emollients, thickeners, preservatives, fragrances, pH regulators and gelling agents.

12. The method according to any one of the preceding claims, wherein the topical antimicrobial composition is a skin antimicrobial aqueous composition.

13. The method according to any one of the preceding claims, wherein the mixture of step a) is carried out until the di(-4-chloro-phenyldigunaido) derivative of formula (1) or the pharmaceutically acceptable salt thereof is completely dissolved in the solvent.

## Patentansprüche

1. Verfahren zur Herstellung einer topischen antimikrobiellen wässrigen Zusammensetzung,
wobei die Zusammensetzung Folgendes umfasst:
50-85 % v/v eines geradkettigen oder verzweigten C₁-C₆-Alkohols,
5-40 % v/v Wasser;
0,001 % bis 20 % w/v von Di(4-chlorphenyldiguanido)-Derivat der Formel (1) 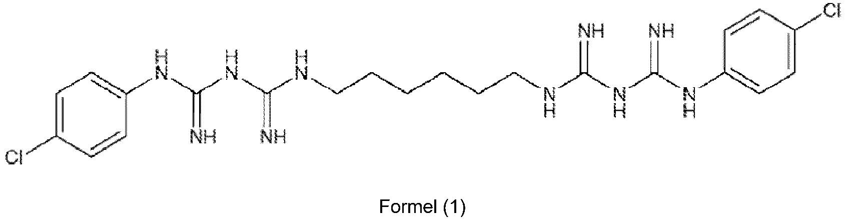
oder eines pharmazeutisch annehmbaren Salzes davon;
von 0,001 % bis 1 % w/v Curcumin;
das ferner Azorubin umfasst,
wobei die v/v- und w/v-Prozentsätze auf das Gesamtvolumen der Zusammensetzung basieren,
und wobei die Zusammensetzung weniger als 2 % THF umfasst;
wobei das Verfahren mindestens die folgenden Schritte umfasst:
a. Bereitstellen einer Lösung von Di(4-chlorphenyldiguanido)-Derivat der Formel (1) oder eines pharmazeutisch annehmbaren Salzes davon in Wasser,
b. Bereitstellen eines Gemisches von Curcumin in Wasser durch Hinzufügen von Wasser zu einem Gefäß mit Curcumin, bis es sich auflöst,
c. Bereitstellen einer separaten Mischung für die zusätzliche Komponente Azorubin durch Hinzufügen von Wasser zu dem Gefäß, das die zusätzliche Komponente enthält, bis es sich auflöst,
d. Hinzufügen zu dem in Schritt a) erhaltenen Gemisch das Gemisch von Schritt b) und das in c) erhaltene Gemisch,
e. Hinzufügen der Wassermenge zu dem Gemisch aus d), die erforderlich ist, um das gewünschte Volumen/Volumenprozent in Bezug auf die Gesamtzusammensetzung zu erreichen, unter Rühren bei einer Temperatur von 10 bis 30 °C bis zur Homogenisierung,
f. Hinzufügen von Alkohol zu dem Gemisch aus e) unter Rühren, bis es homogenisiert ist.

2. Verfahren nach Anspruch 1, wobei das Azorubin in der Zusammensetzung von 0,001 % bis 1 % w/v basierend auf dem Gesamtvolumen der Zusammensetzung vorhanden ist.

3. Verfahren nach einem der vorhergehenden Ansprüche, wobei der geradkettige oder verzweigte C₁-C₆-Alkohol Isopropanol oder Ethanol ist.

4. Verfahren nach einem der vorhergehenden Ansprüche, wobei der Volumenprozentsatz des geradkettigen oder verzweigten C₁-C₆-Alkohols 55-75 % v/v beträgt und/oder der Volumenprozentsatz des Wassers 20-45 % v/v basierend auf dem Gesamtvolumen der Zusammensetzung beträgt.

5. Verfahren nach einem der vorhergehenden Ansprüche, wobei das Di(4-chlorphenyldiguanido)-Derivat der Formel (1) oder das pharmazeutisch annehmbare Salz davon in der Zusammensetzung in einem Bereich von 0,1 bis 10 % w/v basierend auf dem Gesamtvolumen der Zusammensetzung vorhanden ist.

6. Verfahren nach Anspruch 5, wobei das Di(4-chlorphenyldiguanido)-Derivat der Formel (1) oder das pharmazeutisch annehmbare Salz davon in der Zusammensetzung in einem Bereich von 1 bis 4 % w/v, basierend auf dem Gesamtvolumen der Zusammensetzung, vorhanden ist.

7. Verfahren nach einem der vorhergehenden Ansprüche, wobei das pharmazeutisch annehmbare Salz des Di(4-chlorphenyldiguanido)-Derivats der Formel (1) das Gluconat- oder das Hydrochloridsalz ist.

8. Verfahren nach einem der vorhergehenden Ansprüche, wobei die Menge an Curcumin von 0,01 % bis 0,5 % w/v basierend auf dem Gesamtvolumen der Zusammensetzung beträgt.

9. Verfahren nach Anspruch 8, wobei die Menge an Curcumin von 0,01 % bis 0,12 % w/v basierend auf dem Gesamtvolumen der Zusammensetzung beträgt.

10. Verfahren nach einem der vorhergehenden Ansprüche, wobei die Zusammensetzung ferner Hilfsstoffe umfasst, die aus der Liste bestehend aus Emollienzien, Verdickungsmitteln, Konservierungsmitteln, Duftstoffen, pH-Regulatoren und Geliermitteln ausgewählt sind.

11. Verfahren nach einem der vorhergehenden Ansprüche, umfassend:
70 % v/v Isopropanol,
2 % w/v Gluconatsalz des Di(4-chlorphenyldiguanido)-Derivats der Formel (1);
0,050 % w/v Curcumin,
0,01 % w/v Azorubin, und
q.s.p. 100 % v/v Wasser
wobei die Prozentsätze auf dem Gesamtvolumen der Zusammensetzung basieren und gegebenenfalls auch einen oder mehrere pharmazeutisch unbedenkliche Hilfsstoffe umfassen, die ausgewählt sind aus der Gruppe der Emollienzien, Verdickungsmittel, Konservierungsmittel, Duftstoffe, pH-Regulatoren und Geliermittel.

12. Verfahren nach einem der vorhergehenden Ansprüche, wobei die topische antimikrobielle Zusammensetzung eine antimikrobielle wässrige Zusammensetzung für die Haut ist.

13. Verfahren nach einem der vorhergehenden Ansprüche, wobei die Mischung von Schritt a) durchgeführt wird, bis das Di(4-chlorphenyldiguanido)-Derivat der Formel (1) oder das pharmazeutisch annehmbare Salz davon vollständig in dem Lösungsmittel aufgelöst ist.

## Revendications

1. Procédé pour préparer une composition aqueuse antimicrobienne topique,
la composition comprenant :
de 50 à 85 % v/v d'un alcool linéaire ou ramifié en C₁-C₆,
de 5 à 40 % v/v d'eau;
de 0,001 % à 20 % p/v de dérivé di(4-chloro-phényldiguanido) de formule (1) 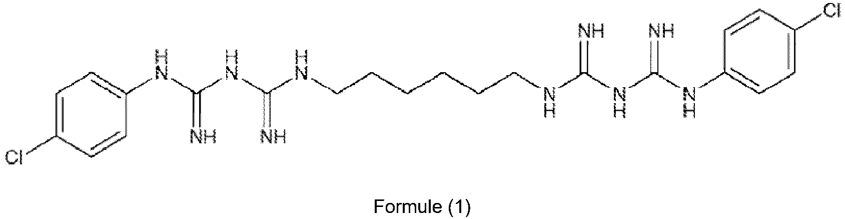
ou un sel pharmaceutiquement acceptable de celui-ci ;
de 0,001 % à 1 % p/v de curcumine ;
qui comprend en outre de l'azorubine,
dans lequel les pourcentages v/v et p/v sont basés sur le volume total de la composition,
et dans lequel la composition comprend moins de 2 % de THF ;
lequel procédé comprend au moins les étapes consistant à :
a. fournir une solution de dérivé di(4-chloro-phényldiguanido) de formule (1) ou un sel pharmaceutiquement acceptable de celui-ci dans l'eau,
b. fournir un mélange de curcumine dans l'eau en ajoutant de l'eau à un récipient avec de la curcumine jusqu'à sa dissolution,
c. fournir un mélange séparé pour le composant supplémentaire azorubine, en ajoutant de l'eau au récipient contenant le composant supplémentaire jusqu'à sa dissolution,
d. ajouter au mélange obtenu à l'étape a) le mélange de l'étape b) et le mélange obtenu à l'étape c),
e. ajouter au mélange de d) la quantité d'eau nécessaire pour atteindre le volume/pourcentage de volume souhaité par rapport à la composition totale, tout en agitant à une température de 10 à 30 °C jusqu'à homogénéisation,
f. ajouter l'alcool au mélange de e) tout en remuant jusqu'à homogénéisation.

2. Procédé selon la revendication 1, dans lequel l'azorubine est présente dans la composition de 0,001 % à 1 % p/v sur la base du volume total de la composition.

3. Procédé selon l'une quelconque des revendications précédentes, dans lequel l'alcool linéaire ou ramifié en C₁-C₆ est l'isopropanol ou l'éthanol.

4. Procédé selon l'une quelconque des revendications précédentes, dans lequel le pourcentage en volume de l'alcool linéaire ou ramifié en C₁-C₆ est de 55 à 75 % v/v et/ou le pourcentage en volume de l'eau est de 20 à 45 % v/v sur la base du volume total de la composition.

5. Procédé selon l'une quelconque des revendications précédentes, dans lequel le dérivé di(4-chloro- phényldiguanido) de la formule (1) ou un sel pharmaceutiquement acceptable de celui-ci est présent dans la composition dans une plage de 0,1 à 10 % p/v sur la base du volume total de la composition.

6. Procédé selon la revendication 5, dans lequel le dérivé di(4-chloro- phényldiguanido) de la formule (1) ou son sel pharmaceutiquement acceptable est présent dans la composition dans une plage de 1 à 4 % p/v, sur la base du volume total de la composition.

7. Procédé selon l'une quelconque des revendications précédentes, dans lequel le sel pharmaceutiquement acceptable du dérivé di(4-chloro-phényldiguanido) de formule (1) est le gluconate ou le sel de chlorhydrate.

8. Procédé selon l'une quelconque des revendications précédentes, dans lequel la quantité de curcumine va de 0,01 % à 0,5 % p/v sur la base du volume total de la composition.

9. Procédé selon la revendication 8, dans lequel la quantité de curcumine va de 0,01 % à 0,12 % p/v sur la base du volume total de la composition.

10. Procédé selon l'une quelconque des revendications précédentes, dans lequel la composition comprend en outre des excipients choisis dans la liste composée d'émollients, d'épaississants, de conservateurs, de parfums, de régulateurs de pH et d'agents gélifiants.

11. Procédé selon l'une quelconque des revendications précédentes, comprenant :
70 % v/v d'isopropanol,
2 % p/v de sel de gluconate de dérivé di(4-chloro-phényldiguanido) de formule (1) ;
0,050 % p/v de curcumine,
0,01 % p/v d'azorubine, et
q.s.p. 100 % v/v d'eau
dans lequel les pourcentages sont basés sur le volume total de la composition, et, comprenant aussi facultativement un ou plusieurs excipients pharmaceutiquement acceptables choisis dans le groupe de : émollients, épaississants, conservateurs, parfums, régulateurs de pH et agents gélifiants.

12. Procédé selon l'une quelconque des revendications précédentes, dans lequel la composition antimicrobienne topique est une composition aqueuse antimicrobienne pour la peau.

13. Procédé selon l'une quelconque des revendications précédentes, dans lequel le mélange de l'étape a) est effectué jusqu'à ce que le dérivé di(4-chloro-phényldiguanido) de formule (1) ou le sel pharmaceutiquement acceptable de celui-ci soit complètement dissous dans le solvant.
